# EUROPÄISCHE PATENTSCHRIFT

(11) **EP 2 810 670 B1**
(45) Veröffentlichungstag und Bekanntmachung des Hinweises auf die Patenterteilung: **07.03.2018**
(21) Anmeldenummer: 14169452.1
(22) Anmeldetag: 22.05.2014
(51) Int. Cl.: A61M 3/02, B05B 7/24

(54) **Medizinische Sprühvorrichtung mit Düse und Verfahren zum Erzeugen eines Sprühkegels**
Medical spraying device with nozzle, and method for producing a spray cone
Dispositif de vaporisation médical équipé d'une buse et procédé de fabrication d'un atomiseur

(30) Priorität: 06.06.2013 DE 102013210539
(43) Veröffentlichungstag der Anmeldung: 10.12.2014
(73) Patentinhaber: Heraeus Medical GmbH, 61273 Wehrheim (DE)
(72) Erfinder: Vogt, Sebastian Dr., 99092 Erfurt (DE)
(74) Vertreter: Schultheiss & Sterzel Patentanwälte PartG mbB

(56) Entgegenhaltungen:
- DE-A1-102011 018 708
- US-A1- 2001 037 095
- US-A1- 2008 311 010

## Beschreibung

Die Erfindung betrifft eine medizinische Sprühvorrichtung zum Ausspülen einer Wunde, insbesondere ein Lavage-System sowie die Verwendung einer solchen Sprühvorrichtung.

Weiterhin ist auch ein Verfahren zum Erzeugen eines Sprühkegels mit einer medizinischen Sprühvorrichtung Gegenstand der Erfindung.

Gegenstand der Erfindung ist somit eine durch Druckgas getriebene medizinische Sprühvorrichtung für die Unfallchirurgie und Orthopädie. Die Sprühvorrichtung kann im Wesentlichen aus Kunststoffen aufgebaut werden und ist bevorzugt für die einmalige Verwendung bestimmt.

Medizinische Sprühvorrichtungen werden im medizinischen Bereich häufig als Lavage-Systeme bezeichnet. Lavage-Systeme werden in der Chirurgie bei Operationen (OPs) in breitem Umfang eingesetzt, um Gewebeareale zu reinigen. Dabei werden häufig physiologische Kochsalzlösung und Ringer-Lösung als Spülflüssigkeiten verwendet. Mit den Lavage-Systemen werden mit den Spülflüssigkeiten Sprühkegel beziehungsweise Sprühstrahlen erzeugt, die auf die zu reinigenden Gewebeareale auftreffen und eine mechanische Reinigungswirkung auf diese Gewebeareale ausüben. Insbesondere bei der Implantation von Gelenkendoprothesen und bei septischen Revisionen sind Lavage-Systeme von wesentlicher Bedeutung (R. M. Sherman et al.: The role of lavage in preventing hemodynamic and blood-gas changes during cemented arthroplasty. J. Bone Joint. Surg. 1983; 65-A: 500-506.; S. J. Breusch et al.: Lavage technique in THA: Jet-lavage Produces Better Cement Penetration Than Syringe-Lavage in the Proximal Femur. J. Arthroplasty. 200; 15(7): 921-927.; R. J. Byrick et al.: High-volume, high pressure pulsatile lavage during cemented arthroplasty. J. Bone Joint Surg. 1989; 81-A: 1331-1336.; J. Christie et al.: Medullary lavage reduces embolic phenomena and cardiopulmonary changes during cemented hemiarthorplasty. J. Bone Joint Surg. 1995; 77-B: 456-459.) Gepulste Lavage-Systeme sind beispielsweise aus der US 4,583,531 A, der US 4,278,078 A und der US 5,542,918 A bekannt. Die DE 698 32 640 T2 offenbart eine Vorrichtung zur Wundirigation, bei der sich Flüssigkeitsstrahlen vor einer Düse treffen. Aus der US 2001/0037095 A1 ist ein Verfahren zum Spülen einer Wunde mit einem zusammenpressbaren Behälter bekannt, wobei eine in dem Behälter enthaltene Spülflüssigkeit durch das Zusammenpressen des Behälters durch eine Mehrzahl von Düsen ausströmen kann, um die Wunde auszuspülen.

Die gegenwärtig im Markt befindlichen Lavage-Systeme werden durch Elektromotoren (zum Beispiel InterPulse® Jet lavage der Stryker GmbH & Co. KG) oder durch Druckluft (zum Beispiel PALAVAGE® der Heraeus Medical GmbH) angetrieben. Bei elektrisch angetriebenen Lavage-Systemen muss jedoch immer ein großer Batterieblock oder Akkumulatorblock mitgeführt werden, der naturgemäß nur eine begrenzte Ladungskapazität hat. Batterie- und Akkumulatorblöcke werden im Hinblick auf ihre Umweltfreundlichkeit kritisch diskutiert. Druckluftgetriebene Lavage-Systeme haben den Vorteil, dass Druckluft im Operationssaal häufig unlimitiert zur Verfügung steht und damit beliebig lange Spülflüssigkeit versprüht werden kann, ohne dass die Energiezufuhr begrenzt ist.

Bei den mit Druckluft oder einem anderen komprimierten Gas getriebenen Systemen wird üblicherweise ein Druckgasmotor zum Antrieb eingesetzt. Bei den meisten Druckgasmotoren für Lavage-Systeme handelt es sich um Lamellen-Druckgasmotoren. Der Druckgasmotor erzeugt eine Drehbewegung, die dann in eine oszillierende, lineare Bewegung umgesetzt wird. Die oszillierende, lineare Bewegung wird genutzt, um jeweils kleinen Volumina des Spülmediums einen Impuls zu verleihen. Dabei wird üblicherweise mindestens eine Membran zwischen dem Antrieb und dem Zulauf der Spülflüssigkeit angeordnet, um die Impulse auf die Spülflüssigkeit übertragen zu können. Es entstehen dadurch Sprühstöße bei hohen Pulszahlen von 2000 bis 3000 Impulsen pro Minute. Das bedeutet, dass der Druckgasmotor sehr präzise gefertigt sein muss, um entsprechend hohe Drehzahlen zu tolerieren. Weiterhin muss eine entsprechend stabile Lagerung vorhanden sein. Aus diesen Gründen ist der Druckgasmotor bei üblichen Druckluft-getriebenen Lavage-Systemen das kostenintensivste Bauteil. Es wird deshalb im Allgemeinen der Druckgasmotor in einem Handgriff aus Metall oder anderen langzeitstabilen Materialien angeordnet, so dass dieses Bauteil mehrfach nach entsprechender Aufbereitung und Sterilisation mehrfach genutzt werden kann.

Nachteilig ist hieran, dass der Aufbau vieler bekannter Lavage-Systeme relativ kompliziert und dadurch kostenaufwendig ist. Durch den Aufbau mit einem Motor besteht immer die Gefahr einer Fehlfunktion des Motors und damit einer Fehlfunktion des Lavage-Systems. Bei einer mehrfachen Anwendung müssen die Lavage-Systeme desinfiziert und vorbereitet werden. Da es bei der Desinfektion zu Fehlern kommen kann, sind eine Verunreinigung der Wunden des Patienten und damit eine komplizierte Infektion nicht auszuschließen. Auch ist die Geräuschentwicklung des Motors im OP-Betrieb störend und für das medizinische Personal belastend.

Die Aufgabe der Erfindung besteht also darin, die Nachteile des Stands der Technik zu überwinden. Insbesondere soll eine möglichst kostengünstig zu fertigende medizinische Sprühvorrichtung bereitgestellt werden, die einen Sprühkegel erzeugt, der zum Debridieren von Wunden geeignet ist.

Die Aufgabe der Erfindung besteht ferner darin, eine einfach zu fertigende medizinische Sprühvorrichtung zu entwickeln, die möglichst einfach aufgebaut ist und die zur einmaligen Verwendung bestimmt sein kann. Der Aufbau der Sprühvorrichtung soll maximal vereinfacht sein und soll aus möglichst wenigen Teilen bestehen. Die Vorrichtung soll möglichst keine Batterien oder Akkumulatoren enthalten. Die Sprühvorrichtung soll weiterhin unabhängig von äußeren Energiequellen ortsunabhängig betrieben werden können. Die zu entwickelnde Sprühvorrichtung soll im Wesentlichen aus kostengünstigen Kunststoffspritzgießteilen gefertigt werden können. Die Vorrichtung soll in der Lage sein, eine medizinische Spülflüssigkeit anzutreiben und somit einen aus Spülflüssigkeitströpfchen gebildeten Strahl beziehungsweise Sprühkegel zu erzeugen, wobei die Spülflüssigkeitströpfchen im Sprühkegel Zufalls-verteilt auftreten sollen. Des Weiteren soll die Vorrichtung möglichst geräuscharm arbeiten.

Die Aufgaben der Erfindung werden gelöst durch eine medizinische Sprühvorrichtung zum Ausspülen einer Wunde, insbesondere Lavage-System, aufweisend ein Flüssigkeitsreservoir für eine medizinische Spülflüssigkeit oder einen Anschluss für ein solches Flüssigkeitsreservoir und eine Druckgaspatrone zur Beaufschlagung der medizinischen Spülflüssigkeit mit einem Druck, wobei die Druckgaspatrone lösbar über eine Druckgasleitung mit dem Flüssigkeitsreservoir verbunden oder verbindbar ist, so dass die Spülflüssigkeit von dem auf die Spülflüssigkeit wirkenden Gasdruck durch eine Düse drückbar ist, um einen Sprühkegel zu erzeugen, wobei die Düse mehrere Öffnungen hat, die in einem Winkel zueinander derart ausgerichtet sind, dass sich die aus den Öffnungen austretenden Spülflüssigkeitsstrahlen in einem Zerstäubungsraum und/oder einer Austrittsöffnung der Düse treffen und dadurch den Sprühkegel aus der zerstäubten Spülflüssigkeit erzeugen, wobei in der Druckgasleitung zwischen dem Anschluss für die Druckgaspatrone und dem Überdruckventil ein Verdampfungsraum zur Verdampfung flüssiger Bestandteile eines verflüssigten Gases aus der Druckgaspatrone angeordnet ist, wobei das verdampfende verflüssigte Gas den Gasdruck erzeugt.

Unmittelbar vor der Düse bedeutet in diesem Zusammenhang, dass der Abstand des Auftreffpunkts der Spülflüssigkeitsstrahlen derart dicht vor der Düse angeordnet ist, dass der Druck beziehungsweise die Strömungsgeschwindigkeit der Spülflüssigkeit ausreicht, um die Spülflüssigkeitsstrahlen zu zerstäuben. Insofern ist der maximale Abstand von der realisierbaren Strömungsgeschwindigkeit der Spülflüssigkeit und damit von dem Druck abhängig, der auf die Spülflüssigkeit wirkt.

Erfindungsgemäß bevorzugt kann vorgesehen sein, dass der Auftreffpunkt oder die Auftreffpunkte der erzeugten Spülflüssigkeitsstrahlen innerhalb eines Abstands von weniger als 2 mm bevorzugt von weniger als 1 mm vor den Öffnungen der Düse liegt oder liegen.

Hierdurch wird auf einfachste Weise eine Vernebelung der Spülflüssigkeit erreicht, ohne dass hierfür ein Motor oder ein bewegliches Teil in der Düse notwendig wäre.

Dabei kann vorgesehen sein, dass die Düse eine zentrale Öffnung zur Erzeugung eines mittigen Hauptstrahls und eine Mehrzahl von um die zentrale Öffnung herum angeordnete äußere Öffnungen aufweist, wobei bevorzugt bezogen auf die Hauptöffnung gegenüberliegende äußeren Öffnungen im gleichen Winkel in Richtung des Hauptstrahls geneigt sind.

Mit dieser Ausführung wird eine gute Vernebelung der Spülflüssigkeit erreicht und gleichzeitig ein kräftiger Sprühstrahl erzeugt.

Ferner kann vorgesehen sein, dass am Flüssigkeitseingang der Düse mindestens zwei Eingangsöffnungen angeordnet sind, so dass die in den Innenraum der Düse eintretende Spülflüssigkeit in mindestens zwei Spülflüssigkeitsströme aufgeteilt ist, die in der Düse derart zu wenigstens zwei Öffnungen geleitet werden, dass sich die mindestens zwei Spülflüssigkeitsstrahlen sich in einem Winkel von mindestens 10° vor der Austrittsöffnung der Düse treffen, bevorzugt sich die Spülflüssigkeitsstrahlen in einem Winkel zwischen 10° und 85° treffen, besonders bevorzugt in einem Winkel zwischen 15° und 45°.

Dadurch übernimmt die Düse alle für das Erzeugen des Sprühkegels wichtigen Funktionen, ohne dass der Aufbau dadurch komplex würde. Die Düse lässt sich einfach aus Kunststoff fertigen.

Mit einer Weiterentwicklung der Erfindung wird auch vorgeschlagen, dass oberhalb der Spülflüssigkeit in dem Flüssigkeitsreservoir ein Gas enthalten ist, über das über die Oberfläche der Spülflüssigkeit ein Druck auf die Spülflüssigkeit ausübbar ist.

Es ist erfindungsgemäß vorgesehen, dass die Einrichtung zur Beaufschlagung der medizinischen Spülflüssigkeit mit einem Druck ein Druckgasreservoir ist, wobei das Druckgasreservoir über eine Druckgasleitung mit dem Flüssigkeitsreservoir verbunden oder verbindbar ist, so dass die Spülflüssigkeit von dem auf die Spülflüssigkeit wirkenden Gasdruck des Druckgasreservoirs durch eine Düse drückbar ist, um den Sprühkegel zu erzeugen.

Durch diesen Aufbau kann auch ein externes Flüssigkeitsreservoir verwendet werden.

Dabei kann vorgesehen sein, dass in der Wandung der Druckgasleitung wenigstens ein Überdruckventil angeordnet ist, das die Druckgasleitung in Abhängigkeit von dem Gasdruck aus der Druckgaspatrone nach außen öffnet und schließt.

Durch die Verwendung des Überdruckventils kann erreicht werden, dass die medizinische Spülflüssigkeit direkt mit dem Gasdruck aus der Druckgasleitung beaufschlagt werden kann und dadurch die Sprühvorrichtung ohne Motor aufgebaut werden kann, ohne dass es zu gefährlichen Überdrucken in der Sprühvorrichtung kommen kann. Hierdurch kann also sichergestellt werden, dass die Sprühvorrichtung auch bei einem Versagen des Druckminderungsventils noch sicher verwendet werden kann. Bevorzugt öffnet sich das Überdruckventil ab einem Grenzdruck zwischen 2 bar und 6 bar.

Mit einer bevorzugten Ausgestaltung der Erfindung wird vorgeschlagen, dass das Flüssigkeitsreservoir von einer elastischen Wandung begrenzt ist, die sich unter Einwirkung des Gasdrucks elastisch verformt, so dass sich bei einer Verringerung des Gasdrucks das Volumen des Flüssigkeitsreservoirs verringert und dabei die Spülflüssigkeit aus dem Flüssigkeitsreservoir durch die Düse herausdrückt.

Durch die Elastizität der Flasche beziehungsweise der Wandungen kann auch dann noch Spülflüssigkeit ausgetragen werden, wenn der Gasdruck plötzlich nachlässt oder schwankt. Des Weiteren ist erfindungsgemäß vorgesehen, dass das Druckgasreservoir eine Druckgaspatrone ist, bevorzugt eine Flüssiggaspatrone ist, besonders bevorzugt eine CO2-Patrone ist, die lösbar mit der Druckgasleitung verbindbar oder verbunden ist, wobei die Druckgaspatrone bevorzugt über ein Öffnungsmittel für die Druckgaspatrone mit der Druckgasleitung verbindbar oder verbunden ist.

Die Anwendung einer Druckgaspatrone bewirkt, dass die Sprühvorrichtung unabhängig von einer externen Druckgasversorgung oder Stromversorgung ist. Alternativ könnte die Sprühvorrichtung auch mit einem Kompressor und einem Stromanschluss oder einem Akkumulator ausgestattet sein.

Bei Ausführungsformen mit Druckgaspatrone ist vorgesehen, dass in der Druckgasleitung zwischen dem Anschluss für die Druckgaspatrone und dem Überdruckventil ein Verdampfungsraum zur Verdampfung flüssiger Bestandteile eines verflüssigten Gases aus der Druckgaspatrone angeordnet ist, wobei das verdampfende verflüssigte Gas den Gasdruck erzeugt.

Hierdurch wird vermieden, dass flüssige Bestanteile des Gases aus der Druckgaspatrone oder direkt dahinter entstehender Schnee oder andere Kondensate tief in die Druckgasleitung vordringen und sich dort störend auswirken.

Ferner kann dabei vorgesehen sein, dass in der Druckgasleitung beim Anschluss für die Druckgaspatrone ein manuell betätigbares Ventil angeordnet ist.

Die Sprühvorrichtung kann dann einfach "scharf" geschaltet werden.

Alternativ zu einer Druckgaspatrone kann vorgesehen sein, dass das Druckgasreservoir an einen Kompressor angeschlossen ist, der bevorzugt über eine flexible Leitung mit der Sprühvorrichtung verbunden ist.

Der Kompressor kann auch Teil eines großflächigen Druckgasnetzes sein, das beispielsweise in einem Krankenhaus zur Verfügung steht.

Mit einer bevorzugten Weiterentwicklung wird vorgeschlagen, dass in der Druckgasleitung ein Druckminderungsventil angeordnet ist, das den Gasdruck begrenzt, der in dem Flüssigkeitsreservoir auf die Spülflüssigkeit wirkt.

Durch die Verwendung des Druckminderungsventils kann sichergestellt werden, dass in der folgenden, dahinter angeordneten Druckgasleitung und damit im Flüssigkeitsreservoir kein zu großer Druck entsteht, der zu einer Zerstörung von Teilen der Druckgasleitung oder der Wandungen des Flüssigkeitsreservoirs beziehungsweise der Flasche führen könnte. Zudem wird so erreicht, dass ein einigermaßen konstanter Druck auf die Spülflüssigkeit drückt und dadurch ein gleichmäßiger Spülflüssigkeitsstrom durch die Düse appliziert werden kann.

Dabei kann vorgesehen sein, dass zwischen dem Druckminderungsventil und dem Druckgasreservoir zumindest ein Sicherungselement angeordnet ist, insbesondere zumindest eine Berstscheibe und/oder zumindest ein Überdruckventil angeordnet ist, das den Gasdruck begrenzt, der auf dem Flüssigkeitsreservoir lastet.

Bei erfindungsgemäßen Spülvorrichtungen kann vorgesehen sein, dass das Flüssigkeitsreservoir über eine Flüssigkeitsleitung mit der Düse verbunden oder verbindbar ist, wobei in der Flüssigkeitsleitung ein manuell betätigbares Ventilelement angeordnet ist, das bevorzugt zur Steuerung des Volumenstroms der Spülflüssigkeit geeignet ist und das besonders bevorzugt mit einem Abzug bedienbar ist.

Hierdurch ist kann der Sprühkegel aufgrund einer manuellen Bedienung erzeugt werden.

Ferner kann vorgesehen sein, dass das Flüssigkeitsreservoir eine Flasche mit einer medizinischen Spülflüssigkeit ist, die über die Druckgasleitung und/oder über die Flüssigkeitsleitung mit der Sprühvorrichtung verbunden oder verbindbar ist, wobei bevorzugt die Flüssigkeitsleitung und die Druckgasleitung durch die gleiche Öffnung der im Betrieb kopfüber angeordneten Flasche münden.

Bevorzugt ist die Flasche über die Druckgasleitung und über die Flüssigkeitsleitung mit der Sprühvorrichtung verbunden oder verbindbar. Mit dieser Ausgestaltung der Erfindung kann eine separate Flasche mit der Spülflüssigkeit verwendet werden, ohne dass die Spülflüssigkeit zuvor in die Vorrichtung eingefüllt werden muss. Die Flaschen können auch leichter gewechselt werden, wenn mehr als der Inhalt einer Flasche zur Behandlung notwendig ist.

Des Weiteren kann bevorzugt vorgesehen sein, dass am Flüssigkeitseingang der Düse mindestens zwei Eingangsöffnungen angeordnet sind, so dass die in den Innenraum der Düse eintretende Spülflüssigkeit in mindestens zwei Spülflüssigkeitsströme aufgeteilt ist, die in der Düse derart zu wenigstens zwei Öffnungen geleitet werden, dass sich die mindestens zwei Spülflüssigkeitsstrahlen sich in einem Winkel von mindestens 10° vor der Austrittsöffnung der Düse treffen, bevorzugt sich die Spülflüssigkeitsstrahlen in einem Winkel zwischen 10° und 85° treffen, besonders bevorzugt in einem Winkel zwischen 15° und 45°.

Mit einer Weiterentwicklung der Erfindung wird vorgeschlagen, dass die Düse als Spitze eines Austragsrohrs vorgesehen ist, wobei bevorzugt das Austragsrohr teleskopartig in axialer Richtung des Austragsrohrs verschiebbar zur Sprühvorrichtung angeordnet ist und/oder das Austragsrohr axial um einen Winkel von mindestens 30° drehbar gelagert ist.

Hierdurch kann die Sprühvorrichtung gut an unterschiedliche Gegebenheiten und Operationssituationen angepasst werden.

Es kann erfindungsgemäß auch vorgesehen sein, dass die Öffnungen der Düse in einem Winkel zueinander derart ausgerichtet sind, dass sich die aus den Öffnungen austretenden Spülflüssigkeitsstrahlen in einem Zerstäubungsraum und/oder einer Austrittsöffnung der Düse treffen.

Durch die Vernebelung beziehungsweise Zerstäubung in dem Zerstäubungsraum wird verhindert, dass sich unzerstäubte Flüssigkeitströpfchen von der Spitze der Düse lösen und unkontrolliert abtropfen. Zudem wird so ein gleichmäßigerer Sprühkegel erreicht.

Mit einer Weiterentwicklung der Erfindung wird auch vorgeschlagen, dass oberhalb der Spülflüssigkeit in dem Flüssigkeitsreservoir ein Gas enthalten ist, über das über die Oberfläche der Spülflüssigkeit ein Druck auf die Spülflüssigkeit ausübbar ist.

Die Aufgaben der Erfindung werden auch gelöst durch die Verwendung einer solchen medizinischen Sprühvorrichtung zum Erzeugen eines Sprühkegels zum Debridement von infiziertem Gewebe.

Ferner werden die Aufgaben der Erfindung auch gelöst durch ein Verfahren zum Erzeugen eines Sprühkegels einer medizinischen Spülflüssigkeit mit einer solchen Sprühvorrichtung bei dem die Spülflüssigkeit durch mehrere Öffnungen einer Düse gedrückt wird und die so erzeugten Spülflüssigkeitsstrahlen mit einer derartigen Strömungsgeschwindigkeit und in einem derartigen Winkel aufeinander geschossen werden, dass die Spülflüssigkeitsstrahlen vor der Düse zerstäuben und einen Sprühkegel bilden.

Dabei kann vorgesehen sein, dass ein Gasdruck aus einer Druckgaspatrone durch eine Druckgasleitung in ein Flüssigkeitsreservoir der medizinischen Spülflüssigkeit geleitet wird und mit dem Gasdruck die Spülflüssigkeit aus dem Flüssigkeitsreservoir durch die Düse gedrückt wird, wobei bei Übersteigen eines Grenzdrucks in der Druckgasleitung zumindest ein Überdruckventil in der Druckgasleitung geöffnet wird und dadurch das Druckgas in die Umgebung strömt und der Druck in der Druckgasleitung reduziert wird, bevorzugt begrenzt wird.

Dabei kann wiederum vorgesehen sein, dass der Gasdruck durch Verdampfen eines Gases aus einer Flüssiggaspatrone, insbesondere einer CO₂-Patron erzeugt wird, wobei bevorzugt das Gas teilweise in einem Verdampfungsraum verflüssigt wird, bevor es zum Überdruckventil geleitet wird.

Es wird auch vorgeschlagen, dass der Gasdruck mit einem Druckminderungsventil in der Druckgasleitung begrenzt wird und der durch das Druckminderungsventil begrenzte Gasdruck durch die Druckgasleitung zu dem Flüssigkeitsreservoir der medizinischen Spülflüssigkeit geleitet wird, wobei bevorzugt der Gasdruck zwischen dem Druckminderungsventil und dem Flüssigkeitsreservoir bei Übersteigen des Grenzdrucks reduziert wird, bevorzugt begrenzt wird.

Der Erfindung liegt die überraschende Erkenntnis zugrunde, dass es mit Hilfe eines einer Düse mit mehreren Öffnungen geeigneter Ausrichtung gelingt, die erzeugten Spülflüssigkeitsstrahlen aus der Düse aufeinander zu schießen, so dass diese vernebelt werden und einen Sprühkegel aus feinem Sprühnebel zu erzeugen. Dadurch kann die Sprühvorrichtung direkt mit Gasdruck betrieben werden, so dass ein Gasdruck unmittelbar auf die medizinische Spülflüssigkeit wirkt und dadurch diese durch die Düse presst, wo die Spülflüssigkeit zu einem Sprühkegel vernebelt wird. Durch die direkte Verwendung des Gasdrucks als Antrieb für die Spülflüssigkeit benötigt das erfindungsgemäße Lavage-System keine Motoren und keine rotierenden oder oszillierenden Teile zum Antrieb der Spülflüssigkeit. Hierdurch wird der Aufbau vereinfacht und somit die Fertigung des Lavage-Systems als nur einmal zu nutzender Wegwerfartikel möglich. Die Fertigung als Einmalartikel hat im medizinischen Bereich den Vorteil, dass keine Desinfektion des Lavage-Systems notwendig ist, bei der Fehler auftreten können und die bei einer Verwendung eines verunreinigten Lavage-Systems zu einer komplizierten Infektion beim Patienten führen können. Zudem kann der gesamte Aufbau sehr kostengünstig gestaltet werden.

Die Erfindung kann dadurch umgesetzt werden, dass am Flüssigkeitseingang der Düse mindestens zwei Öffnungen angeordnet sind, so dass die in den Innenraum der Düse eintretende Spülflüssigkeit in mindestens zwei Spülflüssigkeitsströme aufgeteilt wird, die im Innenraum der Düse so geleitet werden, dass diese sich in einem Winkel von mindestens 10° vor der Austrittsöffnung der Düse treffen. Durch das ineinander strömen beziehungsweise aufeinander schießen der mindestens zwei Spülflüssigkeitsströme wird die Spülflüssigkeit in kleinste Flüssigkeitströpfchen zerstäubt, die sich statistisch verteilt im Sprühkegel bewegen. Dadurch gibt es keine Spülflüssigkeitsstrahlen sondern Zufalls-verteilte einzelne Tröpfchen, so dass das gesamte zu reinigende Gewebeareal mit einzelnen Spülflüssigkeitströpfchen bei entsprechender Einwirkungszeit des Sprühkegels getroffen wird. Dadurch ist eine Reinigungswirkung der Sprühvorrichtung sicher gewährleistet.

Die Richtungsangaben davor oder dahinter beziehen sich auf die Flussrichtung des komprimierten Gases beziehungsweise der medizinischen Spülflüssigkeit.

Bevorzugt weist die medizinische Sprühvorrichtung mindestens ein Ventilelement auf, das den Spülflüssigkeitsstrom zwischen dem Spülflüssigkeitsbehälter und der Düse reguliert, wobei die Düse bevorzugt an einem Austragsrohr angeordnet ist.

Das Ventilelement ist besonders bevorzugt mit einem manuell zu betätigenden Abzug verbunden, der durch mindestens eine Feder im nichtbetätigten Zustand so gehalten wird, dass der Spülflüssigkeitsstrom zwischen dem Spülflüssigkeitsbehälter und der am Austragsrohr angeordneten Düse durch das Ventilelement unterbrochen ist.

Dabei ist es für die medizinische Sprühvorrichtung wesentlich, dass die Gaspatrone ein nicht toxisches Gas enthält, beziehungsweise als Gas für das Gasreservoir ein nicht toxisches Gas verwendet wird. Als Gase, insbesondere für Gaspatronen, kommen Argon, Helium, Distickstoffmonoxid und Kohlendioxid in Betracht. Besonders ist bevorzugt, dass die Gaspatrone flüssiges Kohlendioxid enthält und dass Kohlendioxid als Druckgas bevorzugt wird. Kohlendioxid ist preisgünstig, nicht toxisch und hat den wesentlichen Vorteil, dass es bei Raumtemperatur in verflüssigter Form problemlos in Druckgaspatronen gelagert werden kann. Dadurch ist es möglich, große Gasvolumina in kleinvolumigen Druckgaspatronen bereit zu stellen.

Der gesamte Aufbau bis auf die Düse und gegebenenfalls das Austragsrohr ist erfindungsgemäß bevorzugt in einem Gehäuse angeordnet, wobei das Gehäuse besonders bevorzugt pistolenförmig mit der Düse als Spitze ausgebildet ist. Dadurch kann der medizinische Anwender die Sprühvorrichtung leicht greifen und bedienen.

Eine weitere vorteilhafte Ausgestaltung der Erfindung besteht darin, dass das Austragsrohr in axialer Richtung verschiebbar im Gehäuse angeordnet ist, wobei das Austragsrohr um seine Längsachse um eine Winkel von mindestens 30° drehbar gelagert ist, mindestens einen Zapfen an seinem der Düse entgegengesetzten Rohrende besitzt. Bei einer Ausführung mit Austragsrohr kann erfindungsgemäß bevorzugt vorgesehen sein, dass der Zapfen in eine schlitzförmige Führung des Gehäuses greift und mindestens zwei Aussparungen senkrecht zur schlitzförmigen Führung als Rast für den Zapfen angeordnet sind. Das bedeutet, die Länge des Austragsrohrs kann je nach dem gewünschten Anwendungszweck durch einfaches Heraus- oder Hereinschieben des Austragsrohrs in das Gehäuse variiert werden. Dadurch ist es möglich, ohne Zusatzaustragsrohre, Gewebeareale zu reinigen, bei denen ein kurzes Austragsrohr notwendig ist, wie zum Beispiel bei der Implantation von Knietotalgelenkendoprothesen, und es ist auch nach Herausziehen des Austragsrohrs möglich, Gewebeareale zu reinigen, bei denen ein langes Austragsrohr erforderlich ist, wie zum Beispiel bei der Implantation von Hüftschäften. Durch Drehen des Austragsrohrs um seine Längsachse kann der Zapfen des Austragsrohrs in der gewünschten Position durch Einrasten in die senkrecht zur Führung angeordneten Aussparungen nach dem Prinzip eines Bajonettverschlusses verriegelt werden.

Im Folgenden werden Ausführungsbeispiele der Erfindung anhand von zwei schematisch dargestellten Figuren erläutert, ohne jedoch dabei die Erfindung zu beschränken. Dabei zeigt:
Figur 1: eine schematische Querschnittansicht durch eine erfindungsgemäße medizinische Sprühvorrichtung; und
Figur 2: eine schematische perspektivische Ansicht auf eine Düse und ein teleskopartiges Austragsrohr einer erfindungsgemäßen medizinischen Sprühvorrichtung.

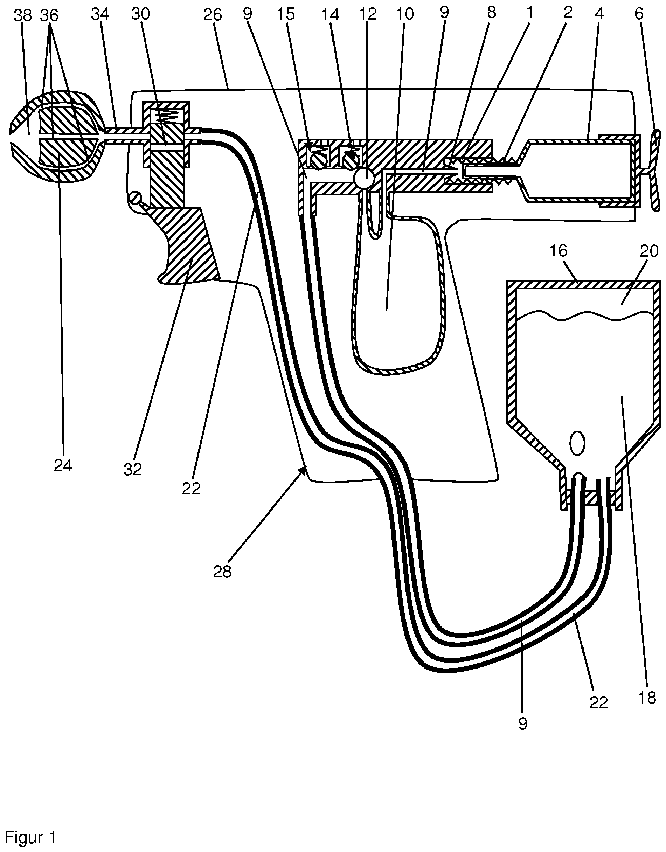

Figur 1 zeigt eine schematische Querschnittansicht durch eine erfindungsgemäße medizinische Sprühvorrichtung. An der Rückseite der Sprühvorrichtung ist eine Halterung mit einem Innengewinde 1 zur Aufnahme eines Außengewindes 2 einer CO₂-Druckgaspatrone 4 vorgesehen. Am Boden der Druckgaspatrone 4 ist ein Drehgriffstück 6 befestigt, um das Eindrehen und Befestigen der Druckgaspatrone 4 in die Halterung der Sprühvorrichtung zu erleichtern.

In der Halterung ist ein Hohldorn 8 angeordnet, der zum Öffnen der Druckgaspatrone 4 dient und der mit einer Druckgasleitung 9 verbunden ist. Beim Eindrehen der Druckgaspatrone 4 wird diese mit einem zum Öffnen vorgesehenen Verschluss auf den Hohldorn 8 aufgedrückt, so dass sich die Druckgaspatrone 4 öffnet und das Druckgas auf der Druckgaspatrone 4 in den Hohldorn 8 und damit in die Druckgasleitung 9 strömt. In der Druckgasleitung 9 ist ein Verdampfungsraum 10 beziehungsweise ein Verdampfungsbehälter 10 angeordnet. Flüssige Bestandteile des CO₂-Gases oder andere schneeartige Kondensate, die aus der Druckgaspatrone in die Druckgasleitung 9 gelangen, werden dort aufgefangen und können dort allmählich verdampfen. Durch diesen Aufbau wird vermieden, dass flüssige oder schneeartige Bestandteile tiefer in die Druckgasleitung 9 vordringen und dort beim Verdampfen zu Unregelmäßigkeiten des Drucks führen.

Alternativ zur Verwendung einer Druckgaspatrone 4 kann auch vorgesehen sein, dass an die Druckgasleitung 9 ein Verbindungsschlauch (nicht gezeigt) einer Druckgasquelle wie beispielsweise einem Kompressor und/oder einer zentralen Druckgasverteilung (nicht gezeigt) angeschlossen ist. Dann kann unter normalen Umständen auch auf den Verdampfungsraum 10 verzichtet werden.

Im weiteren Verlauf der Druckgasleitung 9 ist ein Druckminderungsventil 12 angeordnet, das zur Vereinfachung hier nur als Kreisscheibe dargestellt ist. Mit dem Druckminderungsventil 12 wird der Druck in der weiteren Druckgasleitung 9 auf einem Wert zwischen 1,5 bar und 8 bar begrenzt. Wie dies bei Druckminderungsventile häufig der Fall ist, kann auch bei dem hier verwendeten Druckminderungsventil 12 vorgesehen sein, dass der durch das Druckminderungsventil 12 eingestellte Druck durch eine Stellschraube (nicht gezeigt) eingestellt und manuell verändert werden kann.

Im weiteren Verlauf der Druckgasleitung 9 hinter dem Druckminderungsventil 12 sind zwei Überdruckventile 14, 15 angeordnet, die ab einem Grenzdruck zwischen 2 und 10 bar die Druckgasleitung 9 nach außen in Richtung der Umgebung der Sprühvorrichtung öffnen. Die Überdruckventile 14, 15 sind beispielsweise durch mit Stahlfedern gelagerte Kugeln in einem zylindrischen Hohlraum aufgebaut, wobei die Kugeln durch die Stahlfedern auf einer Kugeloberfläche in Richtung der Druckgasleitung 9 gedrückt werden und dadurch die Druckgasleitung 9 abdichten. Der zylindrische Hohlraum weist zumindest eine Verbindung nach außen an die Umgebung der Sprühvorrichtung auf, die nicht durch die Kugel verdeckt werden kann. Die Überdruckventile 14, 15 dienen dazu, dass in der weiteren Druckgasleitung 9 keine zu hohen Drucke entstehen können, auch wenn das Druckminderungsventil 12 versagt.

Hinter den als blockierbare T-Stücke ausgeformten Überdruckventilen 14, 15 setzt sich die Druckgasleitung 9 als flexibler Schlauch fort, der einen oder mehrere Meter aus der Sprühvorrichtung herausführt und dort über einen Stopfen oder ein anderes Verbindungsmittel mit einer kopfüber aufgehängten Flasche 16 aus einem Kunststoff verbunden ist. In der Flasche 16 ist eine medizinische Spülflüssigkeit 18 zur Behandlung einer Wunde und eine überstehende Gasphase 20 enthalten.

Der Überdruck aus der Druckgasleitung 9 mündet in die Flasche 16 ein und dehnt die überstehende Gasphase 20 und die Flasche 16, sofern diese elastisch ist, aus. Durch den Gasdruck aus der Druckgasleitung 9 und gegebenenfalls auch durch den elastischen Druck der Flasche 16 steht die medizinische Spülflüssigkeit 18 unter Druck und wird durch eine Flüssigkeitsleitung 22 in Richtung einer Düse 24 der Spülvorrichtung gedrückt. Die Flüssigkeitsleitung 22 ist vorliegend ein flexibler Schlauch, der durch den gleichen Stopfen wie der flexible Schlauch der Druckgasleitung 9 in die Flasche 16 eingeführt ist. Der Stopfen dichtet die Flasche 16 ab.

Die meisten Bestandteile der medizinischen Sprühvorrichtung sind in einem Gehäuse 26 aus Plastik angeordnet, das fest mit den restlichen Teilen verbunden ist und das die Form einer Pistole mit einem Pistolengriff 28 hat. Die Flüssigkeitsleitung 22 und der flexible Teile der Druckgasleitung 9, die außerhalb des Gehäuses 26 angeordnet sind, können in einem gemeinsamen, flexiblen Schlauch (nicht gezeigt) eingeschlossen sein, um ein Verheddern der Flüssigkeitsleitung 22 und der externen Druckgasleitung 9 zu verhindern.

Zwischen der Düse 24 und der Flüssigkeitsleitung 22 ist im Inneren des Gehäuses 26 ein manuell bedienbares, mit einer Stahlfeder gefedertes Ventilelement 30 angeordnet, das mit einem drehbar gelagerten Abzug 32 bedienbar ist. In Figur 1 ist das Ventilelement 30 in der geschlossenen Position gezeigt. Hinter dem Ventilelement 30 wird die Flüssigkeitsleitung 22 durch ein Austragsrohr 34 zur Düse 24 geleitet. Es ist bevorzugt, dass das Austragsrohr 34 teleskopartig ausfahrbar ist (nicht gezeigt). Ferner kann die Düse 24 gegen die Achse des Austragsrohrs 34 geneigt und drehbar gelagert sein.

Wenn das Ventilelement 30 über den Abzug 32 bedient wird, wird eine durchgehende Leitung der Spülflüssigkeit 18 aus der Flasche 16 bis in die Düse 24 gebildet. In der Düse 24 sind mehrere Kanäle 36 vorgesehen, so dass der Flüssigkeitsstrom der Spülflüssigkeit 18 innerhalb der Düse 24 in mehrere Flüssigkeitsströme geteilt wird. Die Kanäle 36 sind so geführt, dass die hinter der Düse 24 ausströmenden Spülflüssigkeitsstrahlen (nicht gezeigt) in einem Winkel zwischen 10° und 80° in einem Zerstäubungsraum 38 oder in einer Austrittsöffnung der Düse 24 aufeinander treffen, beziehungsweise aufeinander geschossen werden. Die äußeren Spülflüssigkeitsstrahlen können dabei entlang der Innenwandung des Zerstäubungsraums 38 laufen und treffen im Bereich der zentralen Austrittsöffnung (in Figur 1 links) der Düse 24 auf den zentralen Hauptstrahl. Die aufeinandertreffenden Spülflüssigkeitsstrahlen zerstäuben beziehungsweise vernebeln dabei aufgrund ihrer kinetischen Energie im Zerstäubungsraum 38 zu einem Sprühkegel feiner Spülflüssigkeitströpfchen (nicht gezeigt), der durch die vordere Austrittsöffnung austritt.

Mit der Vorrichtung kann so auf einfachste Weise ein Sprühkegel einer medizinischen Spülflüssigkeit erzeugt werden, ohne dass hierfür ein Motor oder andere sich ständig bewegende Teile benötigt würden. Der Aufbau kann im Wesentlichen aus Kunststoffteilen aufgebaut werden, die durch einfache Spitzgussverfahren herstellbar sind.

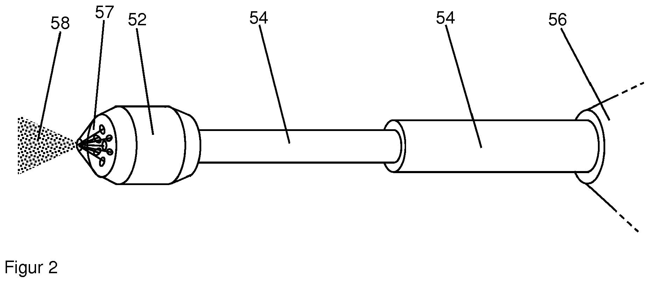

Figur 2 zeigt eine schematische perspektivische Ansicht auf eine Düse 52 und ein teleskopartiges Austragsrohr 54 einer erfindungsgemäßen medizinischen Sprühvorrichtung. Das Austragsrohr 54 ragt aus einem Gehäuse 56 der Sprühvorrichtung. Die restliche Sprühvorrichtung entspricht beispielsweise dem Aufbau nach Figur 1. Die Düse 52 ist außen rotationssymmetrisch geformt.

Im Inneren der Düse 52 wird ein Flüssigkeitsstrom einer durch das Austragsrohr 54 fließenden medizinischen Spülflüssigkeit in sechs Teilströme aufgeteilt, die durch sechs Öffnungen auf der in Figur 2 zu sehenden Vorderfläche der Düse 52 münden. Die Vorderfläche der Düse 52 ist in Richtung des Mittelpunkts der Düse 52 gewölbt. Vor dieser Vorderfläche ist eine konische Kappe aus einem transparenten Kunststoff angeordnet. Der Kunststoff muss nicht transparent sein, dies erleichtert vorliegend aber die Beschreibung der Düsenfunktion mit der Figur 2. Zwischen der konischen Kappe und der Vorderfläche der Düse 52 bildet sich ein Zerstäubungsraum 57. Der Zerstäubungsraum 57 hat eine zentrale Austrittsöffnung, in der sich Flüssigkeitsstrahlen aus den Öffnungen treffen.

Die sieben Flüssigkeitsleitungen im Inneren der Düse 52, sind bis auf die für den mittleren Hauptstrahl im Bereich der Öffnungen in Richtung der Symmetrieachse der äußeren Form der Düse 52 geneigt. Die Neigungen der Leitungen haben gegenüber der Symmetrieachse der äußeren Form der Düse 52 beziehungsweise gegenüber dem zentralen Hauptstrahl alle den gleichen Winkel und die sechs Öffnungen sind symmetrisch um diese Symmetrieachse beziehungsweise den Hauptstrahl herum in gleichen Abständen davon auf der Vorderfläche der Düse 52 verteilt.

Dadurch treffen sieben Spülflüssigkeitsstrahlen (in Figur 2 durch Striche angedeutet), die aus den Öffnungen kommen, alle in einem Bereich (der Austrittsöffnung) aufeinander, zerstäuben beziehungsweise vernebeln in dem Zerstäubungsraum 57 und bilden einen Sprühkegel 58 der medizinischen Flüssigkeit vor der Düse 52, wenn eine Spülflüssigkeit von der Rückseite der Düse 52 aus von der Sprühvorrichtung in die Düse 52 gedrückt wird.

Der teleskopartige Aufbau des Austragsrohrs 54 dient dazu, die Sprühvorrichtung bei unterschiedlichen Einsatzorten universell einsetzbar zu machen. Dazu kann erfindungsgemäß bevorzugt die Neigung der Düse 52 gegenüber dem Austragsrohr 54 einstellbar sein.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Innengewinde
- 2: Außengewinde
- 4: Druckgasreservoir/Druckgaspatrone
- 6: Drehgriffstück
- 8: Hohldorn
- 9: Druckgasleitung
- 10: Verdampfungsraum
- 12: Druckminderungsventil
- 14: Überdruckventil
- 15: Überdruckventil
- 16: Flasche
- 18: Medizinische Spülflüssigkeit
- 20: Überstehende Gasphase
- 22: Flüssigkeitsleitung
- 24: Düse
- 26: Gehäuse
- 28: Pistolengriff
- 30: Ventilelement
- 32: Abzug
- 34: Austragsrohr
- 36: Kanal
- 38: Zerstäubungsraum
- 52: Düse
- 54: Austragsrohr
- 56: Gehäuse
- 57: Zerstäubungsraum
- 58: Sprühkegel

## Patentansprüche

1. Medizinische Sprühvorrichtung zum Ausspülen einer Wunde, insbesondere Lavage-System, aufweisend ein Flüssigkeitsreservoir (16) für eine medizinische Spülflüssigkeit (18) oder einen Anschluss für ein solches Flüssigkeitsreservoir (16), und aufweisend eine Druckgaspatrone (4) zur Beaufschlagung der medizinischen Spülflüssigkeit (18) mit einem Druck, wobei die Druckgaspatrone (4) lösbar über eine Druckgasleitung (9) mit dem Flüssigkeitsreservoir (16) verbunden oder verbindbar ist, so dass die Spülflüssigkeit (18) von dem auf die Spülflüssigkeit (18) wirkenden Gasdruck durch eine Düse (24, 52) drückbar ist, um einen Sprühkegel (58) zu erzeugen, wobei die Düse (24, 52) mehrere Öffnungen hat, die in einem Winkel zueinander derart ausgerichtet sind, dass sich die aus den Öffnungen austretenden Spülflüssigkeitsstrahlen in einem Zerstäubungsraum (38, 57) und/oder einer Austrittsöffnung der Düse (24, 52) treffen und dadurch den Sprühkegel (58) aus der zerstäubten Spülflüssigkeit (18) erzeugen, wobei in der Druckgasleitung (9) zwischen dem Anschluss für die Druckgaspatrone (4) und dem Überdruckventil (14, 15) ein Verdampfungsraum (10) zur Verdampfung flüssiger Bestandteile eines verflüssigten Gases aus der Druckgaspatrone (4) angeordnet ist, wobei das verdampfende verflüssigte Gas den Gasdruck erzeugt.

2. Sprühvorrichtung nach Anspruch 1, **dadurch gekennzeichnet, dass**
die Düse (24, 52) eine zentrale Öffnung zur Erzeugung eines mittigen Hauptstrahls und eine Mehrzahl von um die zentrale Öffnung herum angeordnete äußere Öffnungen aufweist, wobei bevorzugt bezogen auf die Hauptöffnung gegenüberliegende äußeren Öffnungen im gleichen Winkel in Richtung des Hauptstrahls geneigt sind.

3. Sprühvorrichtung nach einem der vorangehenden Ansprüche, **dadurch gekennzeichnet, dass**
in der Wandung der Druckgasleitung (9) wenigstens ein Überdruckventil (14, 15) angeordnet ist, das die Druckgasleitung (9) in Abhängigkeit von dem Gasdruck aus der Druckgaspatrone (4) nach außen öffnet und schließt.

4. Sprühvorrichtung nach einem der vorangehenden Ansprüche, **dadurch gekennzeichnet, dass**
das Flüssigkeitsreservoir (16) von einer elastischen Wandung begrenzt ist, die sich unter Einwirkung des Gasdrucks elastisch verformt, so dass sich bei einer Verringerung des Gasdrucks das Volumen des Flüssigkeitsreservoirs (16) verringert und dabei die Spülflüssigkeit (18) aus dem Flüssigkeitsreservoir (16) durch die Düse (24, 52) herausdrückt.

5. Sprühvorrichtung nach einem der vorangehenden Ansprüche, **dadurch gekennzeichnet, dass**
die Druckgaspatrone (4) eine Flüssiggaspatrone ist, besonders bevorzugt eine CO₂-Patrone ist.

6. Sprühvorrichtung nach einem der vorangehenden Ansprüche, **dadurch gekennzeichnet, dass**
die Druckgaspatrone (4) über ein Öffnungsmittel (8) für die Druckgaspatrone (4) mit der Druckgasleitung (9) verbindbar oder verbunden ist.

7. Sprühvorrichtung nach einem der vorangehenden Ansprüche **dadurch gekennzeichnet, dass**
in der Druckgasleitung (9) ein Druckminderungsventil (12) angeordnet ist, das den Gasdruck begrenzt, der in dem Flüssigkeitsreservoir (16) auf die Spülflüssigkeit (18) wirkt.

8. Sprühvorrichtung nach einem der vorangehenden Ansprüche, **dadurch gekennzeichnet, dass**
das Flüssigkeitsreservoir (16) über eine Flüssigkeitsleitung (22) mit der Düse (24, 52) verbunden oder verbindbar ist, wobei in der Flüssigkeitsleitung (22) ein manuell betätigbares Ventilelement (30) angeordnet ist, das bevorzugt zur Steuerung des Volumenstroms der Spülflüssigkeit (18) geeignet ist und das besonders bevorzugt mit einem Abzug (32) bedienbar ist.

9. Sprühvorrichtung nach einem der vorangehenden Ansprüche, **dadurch gekennzeichnet, dass**
das Flüssigkeitsreservoir (16) eine Flasche (16) mit einer medizinischen Spülflüssigkeit (18) ist, die über die Druckgasleitung (9) und/oder über die Flüssigkeitsleitung (22) mit der Sprühvorrichtung verbunden oder verbindbar ist, wobei bevorzugt die Flüssigkeitsleitung (22) und die Druckgasleitung (9) durch die gleiche Öffnung der im Betrieb kopfüber angeordneten Flasche (16) münden.

10. Sprühvorrichtung nach einem der vorangehenden Ansprüche, **dadurch gekennzeichnet, dass**
am Flüssigkeitseingang der Düse (24, 52) mindestens zwei Eingangsöffnungen angeordnet sind, so dass die in den Innenraum der Düse (24, 52) eintretende Spülflüssigkeit (18) in mindestens zwei Spülflüssigkeitsströme aufgeteilt ist, die in der Düse (24, 52) derart zu wenigstens zwei Öffnungen geleitet werden, dass sich die mindestens zwei Spülflüssigkeitsstrahlen sich in einem Winkel von mindestens 10° vor einer Austrittsöffnung der Düse (24, 52) treffen, bevorzugt sich die Spülflüssigkeitsstrahlen in einem Winkel zwischen 10° und 85° treffen, besonders bevorzugt in einem Winkel zwischen 15° und 45°.

11. Sprühvorrichtung nach einem der vorangehenden Ansprüche, **dadurch gekennzeichnet, dass**
die Düse (24, 52) als Spitze eines Austragsrohrs (34, 54) vorgesehen ist, wobei bevorzugt das Austragsrohr (34, 54) teleskopartig in axialer Richtung des Austragsrohrs (34, 54) verschiebbar zur Sprühvorrichtung angeordnet ist und/oder das Austragsrohr (34, 54) axial um einen Winkel von mindestens 30° drehbar gelagert ist.

12. Sprühvorrichtung nach einem der vorangehenden Ansprüche, **dadurch gekennzeichnet, dass**
oberhalb der Spülflüssigkeit (18) in dem Flüssigkeitsreservoir (16) ein Gas (20) enthalten ist, über das über die Oberfläche der Spülflüssigkeit (18) ein Druck auf die Spülflüssigkeit (18) ausübbar ist.

13. Verfahren zum Erzeugen eines Sprühkegels (58) einer medizinischen Spülflüssigkeit (18) mit einer Sprühvorrichtung nach einem der Ansprüche 1 bis 12 bei dem die Spülflüssigkeit (18) durch mehrere Öffnungen einer Düse (24, 52) gedrückt wird und die so erzeugten Spülflüssigkeitsstrahlen mit einer derartigen Strömungsgeschwindigkeit und in einem derartigen Winkel aufeinander geschossen werden, dass die Spülflüssigkeitsstrahlen vor der Düse (24, 52) zerstäuben und einen Sprühkegel (58) bilden.

14. Verfahren nach Anspruch 13, **dadurch gekennzeichnet, dass**
ein Gasdruck aus einer Druckgaspatrone (4) durch eine Druckgasleitung (9) in ein Flüssigkeitsreservoir (16) der medizinischen Spülflüssigkeit (18) geleitet wird und mit dem Gasdruck die Spülflüssigkeit (18) aus dem Flüssigkeitsreservoir (16) durch die Düse (24, 52) gedrückt wird, wobei bei Übersteigen eines Grenzdrucks in der Druckgasleitung (9) zumindest ein Überdruckventil (14, 15) in der Druckgasleitung (9) geöffnet wird und dadurch das Druckgas in die Umgebung strömt und der Druck in der Druckgasleitung (9) reduziert wird, bevorzugt begrenzt wird.

15. Verfahren nach Anspruch 14, **dadurch gekennzeichnet, dass**
der Gasdruck mit einem Druckminderungsventil (12) in der Druckgasleitung (9) begrenzt wird und der durch das Druckminderungsventil (12) begrenzte Gasdruck durch die Druckgasleitung (9) zu dem Flüssigkeitsreservoir (16) der medizinischen Spülflüssigkeit (18) geleitet wird, wobei bevorzugt der Gasdruck zwischen dem Druckminderungsventil (12) und dem Flüssigkeitsreservoir (16) bei Übersteigen des Grenzdrucks reduziert wird, bevorzugt begrenzt wird.

## Claims

1. Medical spraying device for rinsing of a wound, in particular lavage system, comprising a liquid reservoir (16) for a medical rinsing liquid (18) or a connector for said liquid reservoir (16), and comprising a compressed gas cartridge (4) for application of a pressure to the medical rinsing liquid (18), whereby the compressed gas cartridge (4) is or can be connected detachably to the liquid reservoir (16) via a compressed gas line (9) such that the rinsing liquid (18) can be pushed through a nozzle (24, 52) by the gas pressure acting on the rinsing liquid (18) to generate a spray cone (58), whereby the nozzle (24, 52) has multiple openings that are arranged at an appropriate angle with respect to each other such that the jets of rinsing liquid exiting from the openings meet in an vaporisation space (38, 57) and/or in an exit opening of the nozzle (24, 52) and thus generate the spray cone (58) from the vaporised rinsing liquid (18), whereby an evaporation space (10) for evaporation of liquid components of a liquefied gas from the compressed gas cartridge (4) is arranged in the compressed gas line (9), between the connector for the compressed gas cartridge (4) and the overpressure valve (14, 15), whereby the evaporating liquefied gas generates the gas pressure.

2. Spraying device according to claim 1, **characterised in that**
the nozzle (24, 52) comprises a central opening for generating a central main jet and a plurality of outer openings that are arranged about the central opening, whereby the outer openings that are situated opposite from the main opening preferably are inclined at the same angle in the direction of the main jet.

3. Spraying device according to any one of the preceding claims, **characterised in that**
at least one overpressure valve (14, 15) is arranged in the wall of the compressed gas line (9) and opens and closes the compressed gas line (9) with respect to the outside depending on the gas pressure from the compressed gas cartridge (4).

4. Spraying device according to any one of the preceding claims, **characterised in that**
the liquid reservoir (16) is bordered by an elastic wall that deforms elastically under the action of the gas pressure such that the volume of the liquid reservoir (16) decreases when the gas pressure decreases and, in the process, pushes the rinsing liquid (18) from the liquid reservoir (16) through the nozzle (24, 52).

5. Spraying device according to any one of the preceding claims, **characterised in that**
the compressed gas cartridge (4) is a liquid gas cartridge, particularly preferably a CO₂ cartridge.

6. Spraying device according to any one of the preceding claims, **characterised in that**
the compressed gas cartridge (4) can be or is connected to the compressed gas line (9) by an opening means (8) for the compressed gas cartridge (4).

7. Spraying device according to any one of the preceding claims, **characterised in that**
a pressure reducing valve (12) that limits the gas pressure that acts on the rinsing liquid (18) in the liquid reservoir (16) is arranged in the compressed gas line (9).

8. Spraying device according to any one of the preceding claims, **characterised in that**
the liquid reservoir (16) is or can be connected to the nozzle (24, 52) via a liquid line (22), whereby a valve element (30) that can be operated manually is arranged in the liquid line (22) and preferably is suitable for controlling the volume flow of the rinsing liquid (18) and, particularly preferably, can be operated by means of a trigger (32).

9. Spraying device according to any one of the preceding claims, **characterised in that**
the liquid reservoir (16) is a bottle (16) with a medical rinsing liquid (18) that is or can be connected to the spraying device via the compressed gas line (9) and/or via the liquid line (22), whereby the liquid line (22) and the compressed gas line (9) preferably merge through the same opening of the bottle (16), which, in operation, is arranged upside down.

10. Spraying device according to any one of the preceding claims, **characterised in that**
at least two inlet openings are arranged at the liquid inlet of the nozzle (24, 52) such that the rinsing liquid (18) entering the internal space of the nozzle (24, 52) is divided into at least two rinsing liquid flows, which are guided appropriately in the nozzle (24, 52) toward at least two openings such that the at least two rinsing liquid jets meet at an angle of at least 10° upstream of an exit opening of the nozzle (24, 52), preferably the rinsing liquid jets meet at an angle between 10° and 85°, particularly preferably at an angle between 15° and 45°.

11. Spraying device according to any one of the preceding claims, **characterised in that**
the nozzle (24, 52) is provided as tip of a dispensing tube (34, 54), whereby the dispensing tube (34, 54) preferably is arranged telescope-like in axial direction of the dispensing tube (34, 54) such that it can be shifted with respect to the spraying device and/or the dispensing tube (34, 54) is supported such that it can rotate axially by an angle of at least 30°.

12. Spraying device according to any one of the preceding claims, **characterised in that**
a gas (20), by means of which a pressure can be exerted on the rinsing liquid (18) by means of the surface of the rinsing liquid (18), is contained above the rinsing liquid (18) in the liquid reservoir (16).

13. Method for generating a spray cone (58) of a medical rinsing liquid (18) with a spraying device according to any one of the claims 1 to 12, in which the rinsing liquid (18) is pushed through multiple openings of a nozzle (24, 52) and the rinsing liquid jets thus generated are shot at each other at an appropriate flow rate and at an appropriate angle such that the rinsing liquid jets vaporise upstream of the nozzle (24, 52) and form a spray cone (58).

14. Method according to claim 13, **characterised in that**
a gas pressure from a compressed gas cartridge (4) is fed through a compressed gas line (9) into a liquid reservoir (16) of the medical rinsing liquid (18) and the gas pressure is used to push the rinsing liquid (18) from the liquid reservoir (16) through the nozzle (24, 52) whereby, once a threshold pressure is exceeded in the compressed gas line (9), at least one overpressure valve (14, 15) in the compressed gas line (9) is being opened and thus the compressed gas flows into the surroundings and the pressure in the compressed gas line (9) is being reduced, preferably is being limited.

15. Method according to claim 14, **characterised in that**
the gas pressure is limited by means of a pressure reducing valve (12) in the compressed gas line (9), and the gas pressure limited by the pressure reducing valve (12) is fed through the compressed gas line (9) to the liquid reservoir (16) of the medical rinsing liquid (18), whereby, preferably, the gas pressure is being reduced, preferably is being limited, between the pressure reducing valve (12) and the liquid reservoir (16), once the limit pressure is exceeded.

## Revendications

1. Dispositif de pulvérisation médical pour le rinçage d'une plaie, notamment système de lavage, présentant un réservoir de liquide (16) pour un liquide de rinçage médical (18) ou un raccord pour un tel réservoir de liquide (16), et présentant une cartouche de gaz comprimé (4) pour la sollicitation du liquide de rinçage médical (18) avec une pression, dans lequel la cartouche de gaz comprimé (4) est reliée ou peut être reliée de manière amovible au réservoir de liquide (16) par le biais d'une conduite de gaz comprimé (9) de sorte que le liquide de rinçage (18) peut être poussé par la pression gazeuse agissant sur le liquide de rinçage (18) à travers une buse (24, 52) pour générer un cône de pulvérisation (58), dans lequel la buse (24, 52) a plusieurs ouvertures qui sont dirigées à un angle les unes par rapport aux autres de telle sorte que les jets de liquide de rinçage sortant des ouvertures se rencontrent dans un espace de vaporisation (38, 57) et/ou une ouverture de sortie de la buse (24, 52) et génèrent de ce fait le cône de pulvérisation (58) à partir du liquide de rinçage vaporisé (18), dans lequel un espace d'évaporation (10) pour l'évaporation de constituants liquides d'un gaz liquéfié provenant de la cartouche de gaz comprimé (4) est disposé dans la conduite de gaz comprimé (9) entre le raccord pour la cartouche de gaz comprimé (4) et la soupape de sûreté (14, 15), dans lequel le gaz liquéfié évaporé génère la pression gazeuse.

2. Dispositif de pulvérisation selon la revendication 1, **caractérisé en ce que** la buse (24, 52) présente une ouverture centrale pour la génération d'un jet principal central et une pluralité d'ouvertures extérieures disposées autour de l'ouverture centrale, dans lequel des ouvertures extérieures opposées par rapport à l'ouverture principale sont de préférence inclinées au même angle en direction du jet principal.

3. Dispositif de pulvérisation selon une des revendications précédentes,
**caractérisé en ce que**
au moins une soupape de sûreté (14, 15) qui ouvre vers l'extérieur et ferme la conduite de gaz comprimé (9) en fonction de la pression gazeuse provenant de la cartouche de gaz comprimé (4) est disposée dans la paroi de la conduite de gaz comprimé (9).

4. Dispositif de pulvérisation selon une des revendications précédentes,
**caractérisé en ce que**
le réservoir de liquide (16) est limité par une paroi élastique qui se déforme élastiquement sous l'action de la pression gazeuse de sorte que dans le cas d'une diminution de la pression gazeuse, le volume du réservoir de liquide (16) diminue et pousse ce faisant le liquide de rinçage (18) hors du réservoir de liquide (16) à travers la buse (24, 52).

5. Dispositif de pulvérisation selon une des revendications précédentes,
**caractérisé en ce que**
la cartouche de gaz comprimé (4) est une cartouche de gaz liquide, est de manière particulièrement préférée une cartouche de CO₂.

6. Dispositif de pulvérisation selon une des revendications précédentes,
**caractérisé en ce que**
la cartouche de gaz comprimé (4) peut être reliée ou est reliée à la conduite de gaz comprimé (9) par le biais d'un moyen d'ouverture (8) pour la cartouche de gaz comprimé (4).

7. Dispositif de pulvérisation selon une des revendications précédentes,
**caractérisé en ce que**
une soupape de réduction (12) qui limite la pression gazeuse qui agit sur le liquide de rinçage (18) dans le réservoir de liquide (16) est disposée dans la conduite de gaz comprimé (9).

8. Dispositif de pulvérisation selon une des revendications précédentes,
**caractérisé en ce que**
le réservoir de liquide (16) est relié ou peut être relié à la buse (24, 52) par le biais d'une conduite de liquide (22), dans lequel un élément de soupape (30) pouvant être actionné manuellement qui convient de préférence à la commande du courant volumique du liquide de rinçage (18) et qui peut être manié de manière particulièrement préférée avec un évent (32) est disposé dans la conduite de liquide (22).

9. Dispositif de pulvérisation selon une des revendications précédentes,
**caractérisé en ce que**
le réservoir de liquide (16) est une bouteille (16) avec un liquide de rinçage médical (18) qui est reliée ou peut être reliée au dispositif de pulvérisation par le biais de la conduite de gaz comprimé (9) et/ou par le biais de la conduite de liquide (22), dans lequel la conduite de liquide (22) et la conduite de gaz comprimé (9) débouchent de préférence à travers la même ouverture de la bouteille (16) disposée tête la première en service.

10. Dispositif de pulvérisation selon une des revendications précédentes,
**caractérisé en ce que**
au moins deux ouvertures d'entrée sont disposées à l'entrée de liquide de la buse (24, 52) de sorte que le liquide de rinçage (18) entrant dans l'espace interne de la buse (24, 52) est réparti en au moins deux courants de liquide de rinçage qui sont conduits dans la buse (24, 52) vers au moins deux ouvertures de telle sorte que les au moins deux jets de liquide de rinçage se rencontrent à un angle d'au moins 10° avant une ouverture de sortie de la buse (24, 52), les jets de liquide de rinçage se rencontrent de préférence à un angle compris entre 10° et 85°, de manière particulièrement préférée à un angle compris entre 15° et 45°.

11. Dispositif de pulvérisation selon une des revendications précédentes,
**caractérisé en ce que**
la buse (24, 52) est prévue comme pointe d'un tuyau d'extraction (34, 54), dans lequel le tuyau d'extraction (34, 54) est de préférence disposé de manière télescopique dans la direction axiale du tuyau d'extraction (34, 54) de manière à pouvoir être coulissé par rapport au dispositif de pulvérisation et/ou le tuyau d'extraction (34, 54) est logé de manière à pouvoir être tourné axialement d'un angle d'au moins 30°.

12. Dispositif de pulvérisation selon une des revendications précédentes,
**caractérisé en ce que**
un gaz (20) par le biais duquel une pression peut être exercée sur le liquide de rinçage (18) par le biais de la surface du liquide de rinçage (18) est contenu dans le réservoir de liquide (16) au-dessus du liquide de rinçage (18).

13. Procédé de génération d'un cône de pulvérisation (58) d'un liquide de rinçage médical (18) avec un dispositif de pulvérisation selon une des revendications 1 à 12, dans lequel le liquide de rinçage (18) est poussé à travers plusieurs ouvertures d'une buse (24, 52) et les jets de liquide de rinçage ainsi générés sont tirés les uns sur les autres avec une vitesse d'écoulement telle et à un angle tel que les jets de liquide de rinçage se vaporisent avant la buse (24, 52) et forment un cône de pulvérisation (58).

14. Procédé selon la revendication 13, **caractérisé en ce que** une pression gazeuse provenant d'une cartouche de gaz comprimé (4) est conduite à travers une conduite de gaz comprimé (9) dans un réservoir de liquide (16) du liquide de rinçage médical (18) et le liquide de rinçage (18) est poussé hors du réservoir de liquide (16) à travers la buse (24, 52) avec la pression gazeuse, dans lequel en cas de dépassement d'une pression limite dans la conduite de gaz comprimé (9), au moins une soupape de sûreté (14, 15) est ouverte dans la conduite de gaz comprimé (9) et la pression gazeuse circule de ce fait dans l'environnement et la pression dans la conduite de gaz comprimé (9) est réduite, est de préférence limitée.

15. Procédé selon la revendication 14, **caractérisé en ce que** la pression gazeuse est limitée avec une soupape de réduction (12) dans la conduite de gaz comprimé (9) et la pression gazeuse limitée par la soupape de réduction (12) est conduite à travers la conduite de gaz comprimé (9) vers le réservoir de liquide (16) du liquide de rinçage médical (18), dans lequel la pression gazeuse est de préférence réduite, est de préférence limitée, entre la soupape de réduction (12) et le réservoir de liquide (16) en cas de dépassement de la pression limite.
